# EUROPEAN PATENT APPLICATION

(11) **EP 4 616 862 A1**
(43) Date of publication of application: **17.09.2025**
(21) Application number: 23912994.3
(22) Date of filing: 28.12.2023
(51) Int. Cl.: A61K 31/522, A61P 31/12, A61P 37/02, A23L 33/10, A23K 20/137, A61P 37/00

(54) **ANTIVIRAL COMPOSITION COMPRISING NUCLEOSIDE ANALOGUES DERIVED FROM NUCLEIC ACID AND PHARMACEUTICALLY ACCEPTABLE SALTS THEREOF**

(30) Priority: 29.12.2022 KR 20220189720; 13.06.2023 KR 20230075804
(71) Applicant: CJ CheilJedang Corporation, Seoul 04560 (KR)
(72) Inventor: MOON, Hojin, Seoul 04560 (KR); KIM, Young Nam, Seoul 04560 (KR); KIM, Sung Hun, Seoul 04560 (KR); CHO, Ah Reum, Seoul 04560 (KR)
(74) Representative: Meissner Bolte Partnerschaft mbB
(86) International application number: PCT/KR2023/021896
(87) International publication number: WO 2024/144320

(57) **Abstract**

The present application relates to an antiviral composition, a composition for enhancing immunity, a feed or a feed additive, which comprises a nucleoside analogue derived from nucleotide.

## Description

### [TECHNICAL FIELD]

The present application relates to an antiviral composition, an immunomodulatory composition and a feed composition, which comprise a nucleoside analogue derived from nucleic acid and a pharmaceutically acceptable salt thereof.

### [BACKGROUND ART]

Viruses are infectious pathogens which infect not only animals and plants, but also microorganisms, and cause various kinds of diseases, and disrupt the life cycle of a complete host or cause even death. In particular, in recent years, various animal viruses such as SARS-CoV-2, MERS, Influenza and the like have been infecting humans and causing huge damage for humans extensively all around the world, so it is a situation where they exist as one of great concerns for humanity living in the present age. As a solution to this situation, there may be a way to be free from fear of cross-species infectious viruses derived from animals by controlling viral diseases in animals.

Until a recent date, scientists have been conducting research with infinite passion and efforts to control animal viruses by various methods, but a clear method for perfect control has not been derived yet. For example, a method for using a bait vaccine to a wild animal, commercial vaccination for economical animals and companion animals, and the like have been used, but it is difficult to be expected as a method for exhibiting a practically perfect preventive effect, due to frequent mutations of viruses and slow vaccine development speed, and the like, and in addition, as everyone knows, through paralleling of a vaccine and a therapeutic agent, thorough compliance with quarantine rules, and the like, at least an effect at a level that we expect can be seen.

As a basis for supporting this claim, we already know from experience that it is difficult to completely control animal virus diseases which are prevalent locally or globally with various vaccinations and large-scale quarantine at the national level currently in progress. Many scientists would agree that effective viral disease control could become possible, when a viral therapeutic agent is appropriately used as the last puzzle piece to make an effective virus-related solution in the current situation.

Furthermore, since various viruses occurring constantly according to the laws of nature cannot be prevented in advance, a need for a therapeutic agent or an inhibitor which allows to control viruses afterward rather than a vaccine in a preventive level which is difficult to respond to quickly may arise. However, currently, humanity cannot secure an effective and efficient therapeutic agent against animal viruses, so it can be said that humanity is still suffering from enormous economical and human damage due to various viruses spread from animals.

In this way, in a situation where there is an urgent need for a virus therapeutic agent, first of all, substances for inhibiting virus proliferation against viruses of economical animals and companion animals that are closely related to human life,
African Swine Fever Virus (ASFV), Classical Swine Fever Virus (CSFV), Low pathogenic Avian influenza virus (LPAIV), Canine coronavirus (CCoV), Canine adenovirus (CAV), Canine distemper virus (CDV), Feline parvovirus (FPV), Feline Calicivirus (FCV), Feline infectious peritonitis virus (FIPV, also known as Feline Coronavirus, FCoV) and Foot and Mouth disease virus (FMDV) will be described in the present patent.

### [DISCLOSURE]

### [TECHNICAL PROBLEM]

An object of the present application is to provide an antiviral composition, an immunomodulatory composition or a feed composition which comprises a nucleoside analogue derived from nucleic acid and a pharmaceutically acceptable salt thereof.

### [TECHNICAL SOLUTION]

One of one aspect of the present application may be an antiviral composition comprising nucleoside in a dialdehyde form of inosine, a dialdehyde form of xanthosine, a dialdehyde form of guanosine, or an acyclic diol form of inosine, an acyclic diol form of xanthosine, an acyclic diol form of guanosine.

One of one aspect of the present application may be an immunomodulatory composition comprising nucleoside in a dialdehyde form of inosine, a dialdehyde form of xanthosine, a dialdehyde form of guanosine, or an acyclic diol form of inosine, an acyclic diol form of xanthosine, an acyclic diol form of guanosine.

One of one aspect of the present application may be a medicine, a feed or a feed additive, which comprises the antiviral composition.

Specifically, the composition may be a composition which shows an antiviral effect against at least one virus selected from the group consisting of African Swine Fever Virus (ASFV), Classical Swine Fever Virus (CSFV), Low pathogenic Avian influenza virus (LPAIV), Canine coronavirus (CCoV), Canine adenovirus (CAV), Canine distemper virus (CDV), Feline parvovirus (FPV), Feline Calicivirus (FCV), Feline infectious peritonitis virus (FIPV, also known as Feline Coronavirus, FCoV) and Foot and Mouth disease virus (FMDV).

One of one aspect of the present application may be a method for prevention, improvement, or treatment of a virus comprising administering nucleoside in a dialdehyde form of inosine, a dialdehyde form of xanthosine, a dialdehyde form of guanosine, or an acyclic diol form of inosine, an acyclic diol form of xanthosine, an acyclic diol form of guanosine into a subject.

One of one aspect of the present application may be a method for modulating immunity comprising administering nucleoside in a dialdehyde form of inosine, a dialdehyde form of xanthosine, a dialdehyde form of guanosine, or an acyclic diol form of inosine, an acyclic diol form of xanthosine, an acyclic diol form of guanosine into a subject.

One of one aspect of the present application may be a use for preventing or treating a virus, of nucleoside in a dialdehyde form of inosine, a dialdehyde form of xanthosine, a dialdehyde form of guanosine, or an acyclic diol form of inosine, an acyclic diol form of xanthosine, an acyclic diol form of guanosine.

One of one aspect of the present application may be a use for modulating immunity, of nucleoside in a dialdehyde form of inosine, a dialdehyde form of xanthosine, a dialdehyde form of guanosine, or an acyclic diol form of inosine, an acyclic diol form of xanthosine, an acyclic diol form of guanosine.

### [ADVANTAGEOUS EFFECTS]

The nucleoside analogues derived from nucleic acid according to the present application can be usefully used as an antiviral agent.

### [BEST MODE]

Hereinafter, the present application will be described in detail.

The antiviral or immunomodulatory composition of the present application is a nucleoside analogue in a dialdehyde form or an acyclic diol form, and as one specific aspect, it is characterized by comprising inosine, xanthosine, guanosine in a dialdehyde form or an acyclic diol form, which are derived from nucleic acids represented by the following Chemical formulas 1 to 6, and a pharmaceutically acceptable salt thereof as an active ingredient.

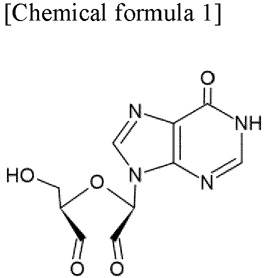

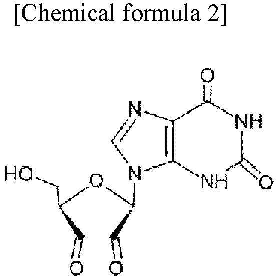

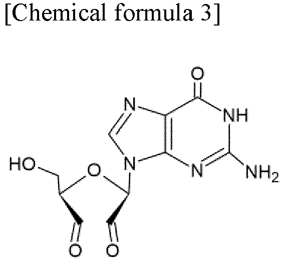

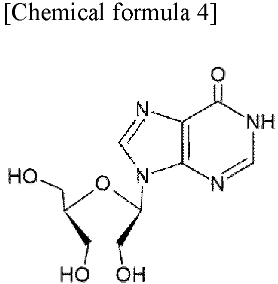

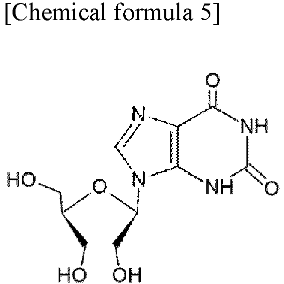

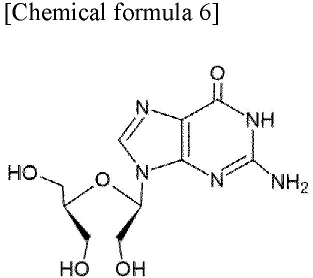

Such an active ingredient, the inosine, xanthosine or guanosine in a dialdehyde form or acyclic diol form can be produced by an optimized process. As one specific embodiment, it may be prepared by the method described in the examples according to the present invention, but not limited thereto.

Then, based on the antiviral composition of 100 parts by weight, the compound represented by Chemical formulas 1 to 6 or a pharmaceutically acceptable salt thereof of 0.0001 part by weight to 20 parts by weight, 0.0001 part by weight to 15 parts by weight, 0.0001 part by weight to 10 parts by weight, 0.0001 to 5 parts by weight, 0.0001 part by weight to 1 part by weight, 0.0001 to 0.5 parts by weight, 0.0001 to 0.1 part by weight, 0.0001 to 0.05 parts by weight, 0.0001 to 0.01 part by weight, 0.0001 to 0.005 parts by weight, 0.0001 to 0.001 part by weight, 0.0001 to 0.0005 parts by weight, 0.001 part by weight to 20 parts by weight, 0.001 part by weight to 15 parts by weight, 0.001 part by weight to 10 parts by weight, 0.001 to 5 parts by weight, 0.001 part by weight to 1 part by weight, 0.001 to 0.5 parts by weight, 0.001 to 0.1 part by weight, 0.001 to 0.05 parts by weight, 0.001 to 0.01 part by weight, 0.001 to 0.005 parts by weight, 0.01 part by weight to 20 parts by weight, 0.01 part by weight to 15 parts by weight, 0.01 part by weight to 10 parts by weight, 0.01 to 5 parts by weight, 0.01 part by weight to 1 part by weight, 0.01 to 0.5 parts by weight, 0.01 to 0.1 part by weight, 0.01 to 0.05 parts by weight, 0.1 part by weight to 20 parts by weight, 0.1 part by weight to 15 parts by weight, 0.1 part by weight to 10 parts by weight, 0.1 to 5 parts by weight, 0.1 part by weight to 1 part by weight, 0.1 to 0.5 parts by weight, 1 to 20 parts by weight, 1 to 15 parts by weight, 1 to 10 parts by weight, 1 to 5 parts by weight, 5 to 20 parts by weight, 5 to 15 parts by weight, 5 to 10 parts by weight, 10 to 20 parts by weight, 10 to 15parts by weight, or 15 to 20 parts by weight may be contained, and therefore, when the compound or pharmaceutically acceptable salt thereof is comprised less than 0.0001 parts by weight, the antiviral effect of the compound is not properly exhibited, and when it is comprised over 20 parts by weight, the increase in the antiviral effect compared to the increase in the compound content is inadequate, so it is not preferable.

The virus is not limited thereto, as long as it is a virus kind, but specifically, it may be a virus derived from a companion animal or industrial animal. Examples of such a companion animal or industrial animal may be a Artiodactyla such as a cow, a pig, a goat, sheep, a deer and the like, fish, an arthropod, a dog, a cat or a bird.

Examples of viruses derived from the Artiodactyla such as a cow, a pig, a goat, sheep, a deer and the like, fish, arthropod, dog, cat or bird, are not limited thereto, but may be at least one selected from the group consisting of African Swine Fever Virus (ASFV), Classical Swine Fever Virus (CSFV), Low pathogenic Avian influenza virus (LPAIV), Canine coronavirus (CCoV), Canine adenovirus (CAV), Canine distemper virus (CDV), Feline parvovirus (FPV), Feline Calicivirus (FCV), Feline infectious peritonitis virus (FIPV, also known as Feline Coronavirus, FCoV) and Foot and Mouth disease virus (FMDV).

Description for representative viruses among viruses of which antiviral efficacy is verified in the present application is as follows.

African swine fever caused by African Swine Fever Virus (ASFV) is a fatal viral hemorrhagic infectious disease, and is a disease causing great economic damage to the pig farming industry as the mortality rate reaches 100% when infected. ASFV has been occurring in Africa since the 1920s, and is present as an endemic disease in most of sub-Saharan Africa, and it also occurred in Europe and South America and the like in the past, and it was eventually eradicated mostly, but in Spain and Portugal, it took 30 years or more to completely eradicate this disease. Since African Swine Fever was introduced in Europe through the Republic of Georgia in 2007, the virus has been widely spread to domestic pigs and wild boars in this region, and thereby, it is currently present as an endemic disease in many Eastern European countries and some regions of the Russian Federation.

ASFV cannot infect humans or other animals and is only susceptible to animals belonging to the Suidae family, and domestic pigs and wild boars are natural hosts, and especially, Warthogs and giant forest hogs, which are wild pigs in the African region, have no clinical symptoms even when infected, so they play a role of a carrier host of the African swine fever virus. Except for pigs, only soft ticks belonging to the Ornithodoros spp. act as a vector playing a role of spreading the disease by carrying this virus and biting pigs or wild boars. Currently, there is no available vaccine or therapeutic agent globally, so it is best to quarantine each country to prevent it from being introduced.

Swine fever is an acute, systemic infectious disease caused by Classical Swine Fever Virus (CSFV), and is a type 1 livestock infectious disease, and upon infection, it shows severe fever reaction, and characteristically shows external symptoms such as spots on skin, and at autopsy, hemorrhagic spots in the bladder and kidney and the like, button-shaped ulcers in the ileocecal colon, spleen hemorrhagic infraction and the like. However, in case of partially immunized pigs, or farms where disease is continuously occurring, these specific symptoms do not appear in most cases.

Preventive means include inoculated of an attenuated live vaccine, and when a vaccine well-stored in a refrigerated state is inoculated according to an appropriate vaccine program, it is possible to effectively prevent the disease. As the disease has been eradicated in many countries such as the United States and the like, neighboring Japan has established and has been pushing ahead an eradication plan. Also in Korea, a vaccine with an excellent effect has been developed and commercialized, so disease outbreaks can be controlled by appropriate vaccination, but it continues to occur as pig farms avoid vaccination for an economic reason.

Avian influenza (AI) is a viral infectious disease caused by infection of pathogenic influenza viruses in wild birds and domesticated poultry, and is divided into non-pathogenic, low pathogenic and highly pathogenic avian influenza as clinical symptoms and pathogenicity are various depending on the pathogenicity of the virus. Among them, highly pathogenic avian influenza (HPAI) is managed as classified as 'type 1 livestock infectious disease' in Korea and 'list A disease' in Office of International Epizootics (OIE), and is subject to thorough surveillance and examination with regard to occurrence in most countries, and therefore, in the event of outbreaks, quarantine measures for eradication such as installing emergency quarantine lines, blocking movement and culling and the like are performed. On the other hand, low pathogenic avian influenza is managed as classified as 'type 2 livestock infectious disease' in Korea, and quarantine means which inhibit occurrence, reduce damage, and reduce spread through vaccination are performed.

All low pathogenic avian influenza viruses occurring in Korea are H9N2 type all, and the first outbreak was reported in 1996, and it shows a low mortality rate, but it causes problems such as reduced egg production, discoloration of egg shells, deterioration of egg quality and reduced feed ingestion (broiler chickens) and the like in layer poultry farms, and thus, it causes serious economic damage. The transmission route of the avian influenza virus may be transmission by droplets, air, water, and the like, and the main transmission route is direct contact with feces. In other words, it is directly transmitted to other chickens by feces stained on boots or clothes of a manager, feed trucks, apparatus, equipment, and egg surface. In 1 g of feces secreted from an infected chicken, viruses which can infect about 1 million chickens are contained. Therefore, in order to prevent transmission, thorough disinfection is very important. For prevention, a H9N2 type inactivated vaccine is commercially available, and it helps minimize economic losses on poultry farms by reducing infection symptoms. On the other hand, there is no commercialized therapeutic agents or preventive drugs, so the reality is relying on simple quarantine.

Canine coronavirus (CCoV) belongs to Coronaviridae, and is a virus having an RNA single strand as genome, and causes canine coronavirus infection. Canine coronavirus infection occurred collectively in the United States in 1971, and at this time, a causative virus was isolated. Many outbreaks of this disease have been reported also in Australia, Japan and Korea, and currently, it is considered one of pathogens of canine viral diarrhea as it is infected as mixed with canine parvovirus infection and further worsens symptoms. It causes an acute viral gastrointestinal infectious disease of which main symptoms are vomiting, diarrhea and dehydration, and both puppies and adult dogs are infected, but particularly, it is caused a lot in puppies and symptoms are distinct. It is susceptible regardless of dogs' breed, age and the like, and it shows rapid transmission and morbidity and in particular, it is developed in group domesticated dogs within a short period of time.

As a preventive measure against canine coronavirus infection, commercially available vaccination has been conducted, but caution for safety is needed. As an example, there has been a problem as encephalitis is caused as a side effect, when a canine distemper attenuated live vaccine and a canine parvovirus vaccine are inoculated together. There is currently no effective therapeutic agent, and symptomatic treatment through early diagnosis may be the most effective. For stabilization and warming of dogs, and minimization of stresses, fluid therapy and antibiotic administration to prevent secondary bacterial infection are used as a part of symptomatic treatment.

Canine adenovirus (CAV) is classified into CAV-1 type which causes canine infectious hepatitis, and CAV-2 type which causes Kennel cough (canine bronchitis or cold). Infectious natural hosts are canine animals such as dogs, foxes and coyotes, and are infected regardless of breed, gender, and age, and are globally spread. Commonly, it is infected through a mouth, and is partially remained in kidney after recovery, and viruses are excreted through urine for about 6~9 months. Therefore, a canine animal infected with this virus plays a role of a reservoir for a long time. After the first infection, it becomes weak after passing through an incubation period of 3~8 days, and has a runny nose and sleep, and shows an abnormal fever reaction. In addition, it also shows gastrointestinal symptoms such as vomiting. In particular, hepatic edema is caused, so abdominal pain is severe, and jaundice symptoms are exhibited in the final stage of hepatitis, and dogs suffering from hepatitis exhibit 'hepatitis blue eye' where eyes turn blue in the recovery stage.

There is no clear treatment method developed yet, and symptomatic treatment (antibiotic treatment to eliminate secondary infection, fluid treatment for in vivo electrolyte balance and dehydration treatment) which allows an infected subject to obtain immunity by himself and be cured is a main method of treatment. As a method of prevention, there is a commercially available vaccine, and a CAV-2 type virus vaccine showing protective activity against CAV-1 type and CAV-2 type is used.

Canine distemper is caused by a canine distemper virus (CDV), and is a canine representative acute, fever viral disease, and after passing through an incubation period of about 3~6 days, it shows rhinitis, fever, severe respiratory symptoms, indigestion, sole hardening, neurological symptoms and the like. In addition, due to strong contagiousness and a high mortality rate, when neurological symptoms are shown, there is almost 100% mortality, and this disease mainly occurs in young dogs under one year of age, but occasionally occurs in older dogs as well. Known transmission routes are feces or urine, nasal secretion, or the like, which is excreted by infected dogs, and the virus is excreted by 60 to 90 days after infection.

As a preventive measure, there is only vaccination for prevention, and the first vaccination is conducted at 2 months of age, and boosting vaccination is carried out at 3~4-week intervals, and additional vaccination is required every year. As current treatment methods, only symptomatic treatment such as administration of a sulfa drug or antibiotic for inhibiting secondary bacterial infection, administration of a glucose agent or electrolyte agent when a dehydration symptom is accompanied, and administration of an adjuvant(immune booster) for rapid recovery, and the like is present.

Feline panleukopenia (panleukopenia, feline infectious enteritis, panleuko) caused by Feline parvo virus (FPV) is fatal for all feline species due to very strong contagiousness and a high mortality rate. The name 'panleukopenia' was named as symptoms of significantly reducing white blood cells are shown in animals infected with this disease. It is mainly infected by being contacted with body fluid, excrements, or the like of an infected animal, but even when is not contact with such a vector, it may be infected also with a flea, bedbug or the like contacted with this vector, and it may be infected not only through bedding and food that have been in contact with this vector, but also clothing and shoes which have been worn. Clinical symptoms generally occur within 4~6 days after exposure, but may appear within 2~14 days, and are not contagious to humans. It is known as a virus which can survive for up to a year at maximum, when an appropriate environment is provided, since causative FPV is structurally very stable, and in addition, even animals recovered from this disease may have the virus in their excretions and the like for up to 6 weeks after recovery.

There are vaccines for prevention, and mainly, are used as a vaccine mixed with various other diseases. In cats, feline panleukopenia has large severity of disease, so a preventive vaccine is recommended to all cats. There is no commercialized therapeutic agent to date, but it has been studied that recombinant interferon omega inhibits proliferation of FPB in an in vitro experiment. As symptomatic treatment, whole blood transfusion is performed to increase the number of white blood cells, and fluid comprising antibiotics and vitamins A, B, C and the like is intravenously injected to prevent sepsis due to dehydration.

Feline Calicivirus (FCV) causes serious acute and chronic respiratory disease in cats, and cats infected with this virus may show acute clinical symptoms or slowly show symptoms. Rarely, there is a case of not showing any clinical symptom, but symptoms appear when stressed or immunity is lowered. Common clinical symptoms include rhinitis, conjunctivitis, stomatitis, gingivitis and glossitis, and occasionally, pneumonia, fever, miscarriage and cystitis are observed. Even after treatment of infection, cats may excrete the virus for several months to several years.

There is no specific therapeutic agent against infection of FCV to date, and antibiotics are administered and an immunomodulating agent is prescribed on a purpose of treating and preventing secondary infection. Treatment of stomatitis is very difficult, and when steroids are prescribed, upper respiratory infection symptoms may further worsen, so close observation is required. As a preventive measure of this disease, a feline calicivirus vaccine has been widely used for the past 20 years, but vaccination cannot prevent 100% of the disease, so there is an urgent need for development of a therapeutic agent. Nevertheless, it is recommended as it can weaken symptoms when infected with FCV.

Feline infectious peritonitis is caused by mutation of Feline Coronavirus (FCoV) infected in cats. It is known that mutations of the virus by various causes are caused in about 10% of cats infected with feline coronavirus, and it proliferates in macrophages, thereby causing systemic disease accompanying immune-mediated vasculitis and purulent granulomatous lesions.

Clinical symptoms caused by feline infectious peritonitis include weight loss, loss of appetite, high fever and the like, and various clinical symptoms are shown depending on the organ to be affected. According to the lesion pattern, it may be divided into an effusive form (wet form) and a non-effusive form (dry form), and feline infectious peritonitis in an effusive form is characterized by fibrinous peritonitis and pleurisy, caused by humoral immune response, and causing effluent in the abdominal cavity, thoracic cavity, or pericardium, and may progress to a systemic disease. The non-effusive form is known that humoral immunity is mainly involved, but cell-mediated immunity is also partially involved therein, and granulomatous lesions occurring in an affected organ cause clinical symptoms. In particular, in case of the non-effusive form, expression of neurological symptoms appears more commonly than the effusive form.

There is a vaccine as a preventive measure, but opinions for the effect of each veterinarian are different. In other words, despite vaccination, peritonitis may be caused, and in addition, there is a problem in safety (the vaccine causes disease), so in most cases, vaccination is not recommended. Unfortunately, it is an incurable disease of which complete treatment and cure are impossible, and symptomatic treatment depending on the symptoms is all. The symptomatic treatment also focuses on alleviating symptoms by immunomodulation. In Japan, there is an opinion of veterinarians, which is that it can be treated by omega interferon, but it is not official method of treatment, and even when used, there are many cases of death, and cost is also very expensive, so it is not used well.

Foot-and-mouth disease virus (FMDV) is a small RNA virus, and this is classified into seven serotypes, that is, A, O, C, Asia1, SAT1, SAT2, SAT3 types, and these major serotypes are divided into about 80 or more subtypes. This virus is a disease which infects animals of which hooves are divided into two such as cows, pigs, goats, sheep, deer and the like (hoofed and even-toed mammal), and its mortality rate is not very high, but it greatly reduces product value of livestock as blisters are formed between lips, togue, nose, and hooves, and loss of appetite, increased body temperature, decreased growth, decreased exercise capacity, and decreased milk production in case of cows. Furthermore, it is very highly contagious, so it is classified as Office of International Epizootics (OIE) List A disease (disease with quick transmissibility and very great economic damage in international trade), and even in Korea, it is designated as type 1 livestock infectious disease.

Currently, as a countermeasure, a method of prevention, a vaccine is only one, but there is inconvenience in that the level of cross-protection between viruses by the vaccine is very week, so different vaccines should be applied depending on the serotype and subtype, and the preventive effect by the vaccine is about 6 months, and does not last very long, so it should be inoculated frequently. In addition, the vaccine produces the same antibodies as the actual antibodies produced by disease, so it is difficult to distinguish infected livestock and vaccinated livestock by a blood test, and thus, when vaccination progresses, it is not possible to be approved as a foot-and-mouth disease-free country, thereby causing problems in the export of related livestock products, and therefore, it may cause great economic problems.

As the pharmaceutically acceptable salt, an acid addition salt formed by pharmaceutically acceptable free acid is useful. As the free acid, inorganic acid and organic acid may be used, and as the inorganic acid, hydrochloric acid, bromic acid, sulfuric acid, sulfurous acid, phosphoric acid, and the like may be used, and as the organic acid, citric acid, maleic acid, fumaric acid, glucosan, methane sulfonate, acetic acid, glycolate, succinic acid, tartaric acid, 4-toluene sulfonate, galacturonic acid, embonic acid, glutamic acid, aspartic acid, and the like may be used. In addition, the pharmaceutical composition containing the nucleoside analogues represented by Chemical formulas 1 to 6 of the present application may comprise not only the pharmaceutically acceptable salts, but also all salts, hydrates and solvates which can be prepared by a common method.

The addition salt according to the present application may be prepared by a common method, and for example, it may be prepared by dissolving a compound selected from the group consisting of compounds represented by Chemical formulas 1 to 6 in a water miscible organic solvent, for example, acetone, methanol, ethanol, or acetonitrile, or the like, and adding an excessive amount of organic acid or adding an acid aqueous solution of inorganic acid, and then immersing or crystallizing. Subsequently, it may be prepared by evaporating the solvent or the excessive amount of acid in this mixture and drying it to obtain an addition salt or suction filtering a precipitated salt.

**In** addition, the composition may be selected from a pharmaceutical composition or a health food composition. In one specific examples, when the antiviral composition is a pharmaceutical composition, it may further comprise at least one additive selected from the group consisting of carriers, excipients, disintegrating agents, sweeteners, covering agents, swelling agents, glidants, flavoring agents, antioxidants, buffer, bacteriostatic agents, diluents, dispersants, surfactants, binders and lubricants, commonly used for preparation of the pharmaceutical composition. Specifically, as the carrier, excipient and diluent, lactose, dextrose, sucrose, sorbitol, mannitol, xylitol, erythritol, maltitol, starch, acacia gum, alginate, gelatin, phosphate calcium, calcium silicate, cellulose, methyl cellulose, microcrystalline cellulose, polyvinyl pyrrolidone, water, methylhydroxy benzoate, propylhydroxy benzoate, talc, magnesium stearate and mineral oils may be used, and solid preparations for oral administration include tablets, pills, powders, granules, capsules and the like, and such solid preparations may be prepared by mixing at least one excipient, for example, starch, calcium carbonate, sucrose or lactose, gelatin, and the like to the composition. Furthermore, in addition to simple excipients, lubricants such as magnesium stearate and talc may be used. Oral liquid preparations include suspensions, oral liquids, emulsions, syrup, and the like, and in addition to water and liquid paraffin which are simple diluents commonly used, various excipients, for example, wetting agents, sweeteners, flavoring agents, preservatives and the like may be comprised. Preparations for parenteral administration include sterilized aqueous solutions, non-aqueous solvents, suspensions, emulsions, freeze-drying agents, suppositories and the like may be used. As the non-aqueous solvents or suspensions, propylene glycol, polyethylene glycol, vegetable oils such as olive oil, injectable esters such as ethyl oleate and the like may be used.

As other embodiment, the pharmaceutical composition may be used as formulated as granules, powders, coated tablets, tablets, pills, capsules, suppositories, gel, syrup, suspensions, emulsions, instillates, or liquid. According to one example of the present application, the pharmaceutical composition may be administered into a subject by a method well-known in the art, for example, by a common method through an oral, intravenous, intra-arterial, intraperitoneal, intramuscular, subcutaneous, intrasternal, dermal, intranasal, inhalation, local, intra-rectal, intraocular, or intradermal route, but not limited thereto.

A specific dose of the compound represented by Chemical formulas 1 to 6 or pharmaceutically acceptable salt thereof may differ depending on the subject's condition and body weight, kind and degree of disease, drug form, administration route and period, and it may be selected appropriately by those skilled in the art.

**In** other embodiment of the present application, the pharmaceutical composition may comprise a compound selected from the group consisting of compounds of Chemical formulas 1 to 6 and pharmaceutically acceptable salts thereof of 0.01 to 90 parts by weight, 0.01 to 50 parts by weight, 0.01 to 10 parts by weight, 0.01 to 5 parts by weight, 0.01 to 1 part by weight, 0.01 to 0.1 part by weight, 0.1 to 90 parts by weight, 0.1 to 50 parts by weight, 0.1 to 10 parts by weight, 0.1 to 5 parts by weight, 0.1 to 1 part by weight, 1 to 90 parts by weight, 1 to 50 parts by weight, 1 to 10 parts by weight, 1 to 5 parts by weight, 10 to 90 parts by weight, 10 to 70 parts by weight, 10 to 50 parts by weight, 10 to 30 parts by weight, or 10 to 20 parts by weight, based on the total pharmaceutical composition of 100 parts by weight, but not limited thereto.

According to one example of the present application, it is not limited thereto, but as clearly mentioned below, when an appropriate amount for each livestock species is under oral administration into an animal, it is absorbed through intestinal epithelial cells, and is introduced into the bloodstream, and spreads to each organ tissue, and in particular, it is delivered/absorbed into cells present in the tissue where a virus proliferates, and the viral proliferation is inhibited in the infected cells by virus challenge, and finally, it can be used as a therapeutic agent of an animal infected by a virus.

In the present application, the subject may be a mammal, a bird, fish, or an arthropod, but not limited thereto. The mammal subject may be a pig, cow, dog, cat or chicken. The fish subject may be flatfish, salmon, sea bream, eel, rockfish, or trout. The arthropod subject may be a shrimp or lobster.

In other embodiment of the present application, the health food may comprise a compound selected from the group consisting of compounds of Chemical formulas 1 to 6 and pharmaceutically acceptable salts thereof by 0.01 to 90 parts by weight, 0.01 to 50 parts by weight, 0.01 to 10 parts by weight, 0.01 to 5 parts by weight, 0.01 to 1 parts by weight, 0.01 to 0.1 part by weight, 0.1 to 90 parts by weight, 0.1 to 50 parts by weight, 0.1 to 10 parts by weight, 0.1 to 5 parts by weight, 0.1 to 1 part by weight, 1 to 90 parts by weight, 1 to 50 parts by weight, 1 to 10 parts by weight, 1 to 5 parts by weight, 10 to 90 parts by weight, 10 to 70 parts by weight, 10 to 50 parts by weight, 10 to 30 parts by weight, or 10 to 20 parts by weight, based on the total health food of 100 parts by weight, but not limited thereto. In other embodiment of the present application, the health food may further comprise at least one additive selected from the group consisting of organic acid, phosphate, antioxidants, lactose casein, dextrin, glucose, sugar and sorbitol. The organic acid is not limited thereto, but may be citric acid, malic acid, adipic acid, or lactose, and the phosphate is not limited thereto, but may be sodium phosphate, potassium phosphate, acidic pyrophosphate or polyphosphate (polymerized phosphate), and the antioxidant is not limited thereto, but may be a natural antioxidant such as polyphenol, catechin, alpha-tocopherol, rosemary extract, licorice extract, chitosan, tannic acid or phytic acid or the like. In other embodiment of the present application, the health food may contain various nutritional supplements, probiotics, vitamins, minerals (electrolytes), flavoring agents such as synthetic flavoring agents and natural flavoring agents, coloring agents and fillers (cheese, chocolate, etc.), pectic acid and salts thereof, alginic acid and salts thereof, organic acids, protective colloidal thickeners, pH adjusting agents, stabilizers, preservatives, glycerin, alcohol, carbonating agents used for carbonated drinks, and the like. According to one example of the present application, the formulation of the health food is not limited thereto, but may be a solid, powder, granule, tablet, capsule, liquid or beverage form. In addition, the health food is not limited thereto, but may be used for preparation of food such as confectionery, sugars, ice cream products, dairy products, meat products, fish meat products, Tofu or jelly, edible oils and fats, noodles, tea, beverages, special nutritional foods, health supplements, seasoning foods, ice, ginseng products, kimchi pickled foods, dried fish and shells, fruits, vegetables, dried products and cut products of fruits or vegetables, fruit juices, vegetable juices, mixed juices thereof, chips, noodles, processed stock raising foods, processed marine foods, processed diary products, fermented oil foods, legume foods, grain foods, fermented microbial foods, confectionery and bakery, spicery, processed meat, acidic beverages, licorice, herbs, and the like.

In addition, the present application provides a feed additive containing a compound selected from the group consisting of compounds of Chemical formulas 1 to 6 and pharmaceutically acceptable salts thereof as an active ingredient.

In one example, the feed additive of the present application may be applied in various forms as same as the antiviral composition. Examples of the preparation forms, are not limited thereto, but include liquids, suspensions, powders, granules, tablets, capsules, pills and the like. In addition, for preparation into the forms, at least one kind of additives, excipients, for example, diluents, glidants, binders, disintegrating agents, sweeteners, stabilizers and preservatives which can be comprised in a common feed additive in addition to the active ingredient may be selected and used, and a flavoring, an adjuvant and the like for providing additional functions may be mixed and used. Specifically, the diluent may be lactose, corn starch, soybean oil, microcrystalline cellulose, or mannitol, and the glidant may be magnesium stearate or talc, and the binder may be polyvinyl pyrrolidone or hydroxypropyl cellulose. In addition, the disintegrating agent may be calcium carboxymethyl cellulose, sodium starch glycolate, polacrilin potassium, or crospovidone, and the sweetener may be white sugar, fructose, sorbitol, aspartame, or nucleic acid (IMP, GMP), and the stabilizer may be sodium carboxymethyl cellulose, betacyclodextrin, white beeswax, or xanthan gum, and the preservative may be methyl parahydroxybenzoate, propyl parahydroxybenzoate, or potassium sorbate.

The feed additive may be selected from the group consisting of mammals, fish, birds or arthropods, specifically, pig, cows, chickens, goats, sheep, horses, fish, shrimps, insects, dogs and cats. Specifically, it may be pigs, cows, chickens, dogs or cats, but not limited thereto. The feed additive can inhibit activity of a virus selected from the group consisting of African Swine Fever Virus (ASFV), Classical Swine Fever Virus (CSFV), Low pathogenic Avian influenza virus (LPAIV), Canine coronavirus (CCoV), Canine adenovirus (CAV), Canine distemper virus (CDV), Feline parvovirus (FPV), Feline Calicivirus (FCV), Feline infectious peritonitis virus (FIPV, also known as Feline Coronavirus, FCoV), and Foot and Mouth disease virus (FMDV).

The feed additive may be fed to a subject such as a mammal, fish, bird or arthropod, specifically, a pig, cow, chicken, goat, sheep, horse, fish, shrimp, insect, dog or cat, by the same regime and dosage as the antiviral composition according to the present application. In addition, the feeding method may be a feeding method well known commonly in the art, for example, orally feeding by mixing with a feed and the like, but not limited thereto.

In one example, the feed additive of the present application may be added at various ratios, for example, such as 0.01 to 300g, 1g to 200g or 10g to 100g (namely, 0.001 % by weight to 30 % by weight, 0.1 % by weight to 20 % by weight or 1 % by weight to 10 % by weight, based on the total feed dry weight) and the like to a feed of 1kg based on the dry weight, in accordance with the above regime and dosage, but not limited thereto.

The antiviral material selected from the compounds of Chemical formulas 1 to 6, and pharmaceutically acceptable salts thereof comprised in the antiviral composition, pharmaceutical composition or feed additive according to the present application shows a mechanism effectively inhibiting viral infection and proliferation by the method of lowering infectivity by inhibiting smooth viral gene replication by reducing sources of viral gene replication through a mechanism of inhibiting functions of inosine monophosphate dehydrogenase (IMPDH) which produces GMP required when a virus invades cells and conducts gene replication, or causing abnormalities in functional proteins of a virus produced as a final product by being inserted during a replication process as a guanosine analogue in a viral gene replication process.

Mechanisms known for several nucleoside analogues, for example, well-known efficacy mechanisms such as an immunomodulatory effect, an increase in expression of interferon stimulating factors, inhibition of mechanisms of RNA polymerase of a virus, and the like, may also be mechanisms selected from the compounds of Chemical formulas 1 to 6, and pharmaceutically acceptable salts thereof comprised in the antiviral composition, pharmaceutical composition or feed additive according to the present application.

### [MODE FOR INVENTION]

Hereinafter, in order to help understanding of the present application, it will be described in detail by examples. However, the following examples illustrate the contents of the present application only, but the scope of the present application is not limited by the following examples. The examples of the present application are provided to describe the present application more completely to those skilled in the art.

### [Example]

### Preparative example 1: Preparation method of nucleoside analogues using inosine monophosphate (IMP), xanthosine monophosphate (XMP), and guanosine monophosphate (GMP)

### 1-1. Preparation method of nucleoside in a dialdehyde form

For oxidative cleavage, IMP, XMP, or GMP dissolved in water (prepared and supplied from CJ CheilJedang) was prepared using periodate (NaIO₄) as a catalyst and using an organic solvent, and then was filtered using filter paper and permeates were purified with anion exchange resin (WA30) and then freeze-dried to obtain final products (Maria Meurillon et al., 2014 Eur. J. Med. Chem. 77:18-37).

### 1-2. Preparation method of nucleoside in an acyclic diol form

For oxidative cleavage, IMP, XMP, or GMP dissolved in water (prepared and supplied from CJ CheilJedang) was prepared using periodate (NaIO₄) as a catalyst and using an organic solvent, and then was filtered using filter paper and permeates were reduced by adding sodium borohydride (NaBH₄), and then freeze-dried to obtain final products (Maria Meurillon et al., 2014 Eur. J. Med. Chem. 77:18-37).

### Example 1: African Swine Fever Virus (ASFV)

In order to evaluate viral infection and inhibitory efficacy of inosine, xanthosine, and guanosine in a dialdehyde form and an acyclic diol form against African Swine Fever Virus (ASFV, China/2018/AnhuiXCGQ), swine alveolar macrophages (PAM cell line) were seeded in a 48 well plate by 0.5x10⁵ cells/well, and then cultured for one day. Next day, ASFV of 1 MOI and inosine, xanthosine, and guanosine in a dialdehyde form and an acyclic diol form were simultaneously treated into the cells, and after 2 days, DNA was extracted from the infected cells using a Viral gene (DNA/RNA) extraction kit (iNtRON, 101410754). For the extracted DNA, using ASFV-specific primers (F-AGTTCGGATGTCACAACGCT, R-ACTGGTTCCCTCCACCGATA) and realtime RT-qPCR experimental method (95°C, 5 min 1 cycle, 95°C, 10 sec, 65°C, 30 sec, 56°C, 60 sec, 40 cycles), the antiviral efficacy of the materials was verified.

As could be seen in [Table 1], it could be confirmed that infection and proliferation of ASFV were inhibited, when inosine, xanthosine and guanosine in a dialdehyde form and an acyclic diol form were treated into cells. The guanosine in a dialdehyde form showed the best efficacy (IC₅₀: 79.0 µM), and the antiviral effect was shown in the order of inosine and xanthosine. In addition, also in the materials in the acyclic diol form, the antiviral efficacy against ASFV was shown in the order of guanosine, inosine and xanthosine.

**[Table 1]**

| ASFV infection inhibition concentration, cytotoxicity and selectivity index by inosine, xanthosine and guanosine in a dialdehyde form and an acyclic diol form | | | |
|---|---|---|---|
| Classification | IC₅₀ | CC₅₀ | SI |
| Dialdehyde-Inosine | < 96.6 uM (±1.26) | > 10000 uM | > 103.5 |
| Dialdehyde-Xanthosine | < 110.0 uM (±1.1) | > 10000 uM | > 90.9 |
| Dialdehyde-Guanosine | < 79.0 uM (±1.0) | > 6000 uM | > 75.9 |
| Acyclic diol-Inosine | < 353.0 uM (±1.1) | > 10000 uM | > 28.3 |
| Acyclic diol-Xanthosine | < 1165 uM (±1.1) | > 10000 uM | > 8.6 |
| Acyclic diol-Guanosine | < 270.0 uM (±1.6) | > 10000 uM | > 37.0 |

### Example 2: Classical Swine Fever Virus (CSFV)

In order to evaluate viral infection and inhibitory efficacy of inosine, xanthosine, and guanosine in a dialdehyde form and an acyclic diol form against Classical Swine Fever Virus (CSFV), swine kidney cells (PK15 cell line) were seeded in a 96 well plate by 1x10⁴ cells/well, and then cultured for one day, and then, CSFV of 100 TCID₅₀/well and inosine, xanthosine, and guanosine in a dialdehyde form and an acyclic diol form were simultaneously treated into the cells, and after 2 days, RNA was isolated from the infected cells using a commercialized viral RNA extraction kit (Ribospin^{™} vRD II). For the isolated RNA, using CSFV-specific primers (F-CTCTGGTCAGGGTGCTCAAG, R-GAGGGACTGTGCAACCATCA) and realtime RT-qPCR experimental method (95°C, 15 min 1 cycle, 95°C, 20 sec, 58°C, 40 sec, 40 cycles), the antiviral efficacy of the materials was verified.

As could be seen in [Table 2], the inhibitory efficacy of infection and proliferation of CSFV by inosine, xanthosine and guanosine in a dialdehyde form and an acyclic diol form could be confirmed. The xanthosine in a dialdehyde form showed the best efficacy (IC₅₀: 156.7 µM), and the antiviral effect was shown in the order of guanosine and inosine. Additionally, in the acyclic diol form, the IC₅₀ value was shown in the order of inosine, xanthosine and guanosine, and a relatively higher IC₅₀ value compared to the dialdehyde form was shown.

**[Table 2]**

| CSFV infection inhibition concentration, cytotoxicity and selectivity index by inosine, xanthosine and guanosine in a dialdehyde form and an acyclic diol form | | | |
|---|---|---|---|
| Classification | IC₅₀ | CC₅₀ | SI |
| Dialdehyde-Inosine | < 238.3 uM (±85.3) | > 10000 uM | > 42.0 |
| Dialdehyde-Xanthosine | < 156.7 uM (±65.2) | > 10000 uM | > 63.8 |
| Dialdehyde-Guanosine | < 165.2 uM (±15.6) | > 6000 uM | > 36.1 |
| Acyclic diol-Inosine | < 1754 uM (±302.5) | > 10000 uM | > 5.7 |
| Acyclic diol-Xanthosine | < 2960 uM (±371.5) | > 10000 uM | > 3.4 |
| Acyclic diol-Guanosine | < 3608 uM (±868.5) | > 10000 uM | > 2.8 |

### Example 3: Low pathogenic avian influenza virus (LPAIV)

In order to evaluate viral infection and inhibitory efficacy of inosine, xanthosine, and guanosine in a dialdehyde form and an acyclic diol form against Low pathogenic avian influenza virus (LPAIV, H9N2), canine kidney cells (MDCK cell line) were seeded in a 96 well plate by 1.4x10⁴ cells/well, and then cultured for one day. Next day, LPAIV of 20 TCID₅₀/well and inosine, xanthosine, and guanosine in a dialdehyde form and an acyclic diol form were simultaneously treated into the cells, and after 2 days, RNA was isolated from the infected cells using a commercialized viral RNA extraction kit (Ribospin^{™} vRD II). For the extracted RNA, using LPAIV-specific primers (F-GCTAGGCAGATGGTACAGGC, R-TGCACTCCCATCCGTTTCTG) and realtime RT-qPCR experimental method (95°C, 15 min 1 cycle, 95°C, 20 sec, 58°C, 40 sec, 40 cycles), the antiviral efficacy of the materials was verified.

As could be seen in [Table 3], the guanosine in a dialdehyde form showed the most excellent efficacy (IC₅₀: 102.2 µM), and the inhibitory efficacy of viral infection and proliferation was confirmed in the order of xanthosine and inosine. In addition, in case of the materials in an acyclic diol form, antiviral efficacy against LPAIV was shown in the order of guanosine, inosine and xanthosine.

**[Table 3]**

| LPAIV infection inhibition concentration, cytotoxicity and selectivity index by inosine, xanthosine and guanosine in a dialdehyde form and an acyclic diol form | | | |
|---|---|---|---|
| Classification | IC₅₀ | CC₅₀ | SI |
| Dialdehyde-Inosine | < 267.7 uM (±131.0) | > 10000 uM | > 37.4 |
| Dialdehyde-Xanthosine | < 221.8 uM (±20.6) | > 10000 uM | > 45.1 |
| Dialdehyde-Guanosine | < 102.2 uM (±2.0) | > 6000 uM | > 58.7 |
| Acyclic diol-Inosine | < 1811 uM (±101.8) | > 10000 uM | > 5.5 |
| Acyclic diol-Xanthosine | < 2005 uM (±697.9) | > 10000 uM | > 5.0 |
| Acyclic diol-Guanosine | < 1336 uM (±246.8) | > 10000 uM | > 7.5 |

### Example 4: Canine coronavirus (CCoV)

In order to evaluate viral infection and inhibitory efficacy of inosine, xanthosine, and guanosine in a dialdehyde form and an acyclic diol form against Canine coronavirus (CCoV), canine fibroblasts (A-72 cell line) were seeded in a 96 well plate by 1.4x10⁴ cells/well, and then cultured for one day. Next day, CCoV of 50 TCID₅₀/well and inosine, xanthosine, and guanosine in a dialdehyde form and an acyclic diol form were simultaneously treated into the cells, and after 2 days, RNA was extracted from the infected cells using a commercialized viral RNA extraction kit (Ribospin^{™} vRD II). For the extracted RNA, using CCoV-specific primers (F-TGAAGGTGTGCCAACTGGTGT, R-GCCCATCCTGTCGCACTACT) and realtime RT-qPCR experimental method (95°C, 15 min 1 cycle, 95°C, 20 sec, 58°C, 40 sec, 40 cycles), the antiviral efficacy of the materials was verified.

As could be seen in [Table 4], the inosine and xanthosine in a dialdehyde form showed excellent efficacy (IC₅₀: 45.1 µM and 43.0 µM), and the guanosine showed the IC₅₀ value of 109.7 µM. Additionally, in case of the materials in an acyclic diol form, antiviral efficacy against CCoV could be observed in the order of inosine, guanosine and xanthosine.

**[Table 4]**

| CCoV infection inhibition concentration, cytotoxicity and selectivity index by inosine, xanthosine and guanosine in a dialdehyde form and an acyclic diol form | | | |
|---|---|---|---|
| Classification | IC₅₀ | CC₅₀ | SI |
| Dialdehyde-Inosine | < 45.1 uM (±0.8) | > 10000 uM | > 221.7 |
| Dialdehyde-Xanthosine | < 43.0 uM (±0.3) | > 10000 uM | > 232.6 |
| Dialdehyde-Guanosine | < 109.7 uM (±12.1) | > 6000 uM | > 54.7 |
| Acyclic diol-Inosine | < 1273 uM (±112.4) | > 10000 uM | > 7.9 |
| Acyclic diol-Xanthosine | < 2603 uM (±265.2) | > 10000 uM | > 3.8 |
| Acyclic diol-Guanosine | < 2244 uM (±178.9) | > 10000 uM | > 4.5 |

### Example 5: Canine adenovirus (CAV)

In order to evaluate viral infection and inhibitory efficacy of inosine, xanthosine, and guanosine in a dialdehyde form and an acyclic diol form against Canine adenovirus (CAV), monkey kidney cells (Vero cell line) were seeded in a 96 well plate by 2x10⁴ cells/well, and then cultured for one day. Next day, CAV of 100 TCID₅₀/well and inosine, xanthosine, and guanosine in a dialdehyde form and an acyclic diol form were simultaneously treated into the cells, and after 2 days, DNA was extracted from the infected cells using a commercialized viral gene (DNA/RNA) extraction kit (iNtRON, 101410754). For the extracted DNA, using CAV-specific primers (F-CGCTGAACATTACTACCTTGTC, R-GCAGAGTCTAGAACAAATGGC) and realtime RT-qPCR experimental method (95°C, 5 min 1 cycle, 95°C, 15 sec, 60°C, 30 sec, 40 cycles), the antiviral efficacy of the materials was verified.

As could be seen in [Table 5], the inhibitory efficacy of infection and proliferation of CAV by inosine, xanthosine and guanosine in a dialdehyde form and an acyclic diol form could be confirmed. The guanosine in a dialdehyde form showed the most excellent efficacy (IC₅₀: 85.9 µM), and the antiviral effect was shown in the order of xanthosine and inosine. Additionally, in the acyclic diol form, the antiviral efficacy was shown in the order of xanthosine, inosine and guanosine, and a relatively higher IC₅₀ value compared to the dialdehyde form was shown.

**[Table 5]**

| CAV infection inhibition concentration, cytotoxicity and selectivity index by inosine, xanthosine and guanosine in a dialdehyde form and an acyclic diol form | | | |
|---|---|---|---|
| Classification | IC₅₀ | CC₅₀ | SI |
| Dialdehyde-Inosine | < 325.6 uM (±119.3) | > 10000 uM | > 30.7 |
| Dialdehyde-Xanthosine | < 129.5 uM (±1.4) | > 10000 uM | > 77.2 |
| Dialdehyde-Guanosine | < 85.9 uM (±0.3) | > 6000 uM | > 69.8 |
| Acyclic diol-Inosine | < 1779 uM (±86.3) | > 10000 uM | > 5.6 |
| Acyclic diol-Xanthosine | < 1353 uM (±157.0) | > 10000 uM | > 7.4 |
| Acyclic diol-Guanosine | < 4133 uM (±755.9) | > 10000 uM | > 2.4 |

### Example 6: Canine distemper virus (CDV)

In order to evaluate viral infection and inhibitory efficacy of inosine, xanthosine, and guanosine in a dialdehyde form and an acyclic diol form against Canine distemper virus (CDV), monkey kidney cells (Vero cell line) were seeded in a 96 well plate by 2x10⁴ cells/well, and then cultured for one day. Next day, CDV of 100 TCID₅₀/well and inosine, xanthosine, and guanosine in a dialdehyde form and an acyclic diol form were simultaneously treated into the cells, and after 3 days, RNA was extracted from the infected cells using a commercialized viral RNA extraction kit (Ribospin^{™} vRD II). For the extracted RNA, using CDV-specific primers (F-GCTTACTTCAGACTCGGGCAAGAAATGGTTA, R-CAGTAGCTCGAATTGTCCGGTCCTCTGTTGT) and realtime RT-qPCR experimental method (95°C, 10 min 1 cycle, 95°C, 15 sec, 60°C, 30 sec, 72°C, 30 sec, 40 cycles), the antiviral efficacy of the materials was verified.

As could be seen in [Table 6], the xanthosine in a dialdehyde form showed the most excellent efficacy (IC₅₀: 79.9 µM), and the inhibitory efficacy of viral infection and proliferation was confirmed in the order of inosine and guanosine. The materials in the acyclic diol form showed the antiviral efficacy at the IC₅₀ value at a relatively high concentration compared to the materials in the dialdehyde form, and the efficacy ranking was confirmed as xanthosine, guanosine and inosine.

**[Table 6]**

| CDV infection inhibition concentration, cytotoxicity and selectivity index by inosine, xanthosine and guanosine in a dialdehyde form and an acyclic diol form | | | |
|---|---|---|---|
| Classification | IC₅₀ | CC₅₀ | SI |
| Dialdehyde-Inosine | < 106.5 uM (±8.9) | > 10000 uM | > 93.9 |
| Dialdehyde-Xanthosine | < 79.9 uM (±21.8) | > 10000 uM | > 125.1 |
| Dialdehyde-Guanosine | < 129.0 uM (±39.5) | > 6000 uM | > 46.5 |
| Acyclic diol-Inosine | < 2698 uM (±206.5) | > 10000 uM | > 3.7 |
| Acyclic diol-Xanthosine | < 2046 uM (±545.2) | > 10000 uM | > 4.9 |
| Acyclic diol-Guanosine | < 2121 uM (±125.2) | > 10000 uM | > 4.7 |

### Example 7: Feline parvovirus (FPV)

In order to evaluate viral infection and inhibitory efficacy of inosine, xanthosine, and guanosine in a dialdehyde form and an acyclic diol form against Feline parvovirus (FPV), feline kidney cells (CRFK cell line) were seeded in a 48 well plate by 3x10⁴ cells/well, and then cultured for one day. Next day, FPV of 100 TCID₅₀/well and inosine, xanthosine, and guanosine in a dialdehyde form and an acyclic diol form were simultaneously treated into the cells, and after 2 days, DNA was extracted from the infected cells using a commercialized viral gene (DNA/RNA) extraction kit (iNtRON, 101410754). For the extracted DNA, using FPV-specific primers (F-AGAGCATTGGGCTTACCACC, R-CCCCATTTGAGTTACACCACG) and realtime RT-qPCR experimental method (95°C, 15 min 1 cycle, 95°C, 20 sec, 58°C, 30 sec, 72°C, 30 sec, 40 cycles), the antiviral efficacy of the materials was verified.

As could be seen in [Table 7], it could be confirmed that infection and proliferation of FPV were inhibited, when inosine, xanthosine and guanosine in a dialdehyde form and an acyclic diol form were treated into cells. The xanthosine in a dialdehyde form showed the most excellent efficacy (IC₅₀: 31.0 µM), and excellent effects were confirmed in the order of guanosine and inosine. In addition, in the acyclic diol form, the IC₅₀ value could be confirmed in the order of guanosine, inosine and xanthosine.

**[Table 7]**

| FPV infection inhibition concentration, cytotoxicity and selectivity index by inosine, xanthosine and guanosine in a dialdehyde form and an acyclic diol form | | | |
|---|---|---|---|
| Classification | IC₅₀ | CC₅₀ | SI |
| Dialdehyde-Inosine | < 52.0 uM (±1.6) | > 10000 uM | > 192 |
| Dialdehyde-Xanthosine | < 31.0 uM (±7.9) | > 8000 uM | > 258 |
| Dialdehyde-Guanosine | < 38.0 uM (±21.8) | > 6000 uM | > 158 |
| Acyclic diol-Inosine | < 1867 uM (±241.7) | > 10000 uM | > 5.4 |
| Acyclic diol-Xanthosine | < 5750 uM (±246.1) | > 10000 uM | > 1.7 |
| Acyclic diol-Guanosine | < 530 uM (±594.0) | > 10000 uM | > 1.9 |

### Example 8: Verification of inhibitory efficacy of infection against Feline Calicivirus (FCV)

In order to evaluate viral infection and inhibitory efficacy of inosine, xanthosine, and guanosine in a dialdehyde form and an acyclic diol form against Feline Calicivirus (FCV), feline kidney cells (CRFK cell line) were seeded in a 48 well plate by 3x10⁴ cells/well, and then cultured for one day. Next day, FCV of 100 TCID₅₀/well and inosine, xanthosine, and guanosine in a dialdehyde form and an acyclic diol form were simultaneously treated into the cells, respectively, and after 24 hours, RNA was extracted from the infected cells using a commercialized viral RNA extraction kit (Ribospin^{™} vRD II). For the extracted RNA, using FCV-specific primers (F-GCAAAGATCCGGCTTGCCTC, R-CGCTGTTGACCAAGTGCAGC) and realtime RT-qPCR experimental method (95°C, 15 min 1 cycle, 95°C, 20 sec, 58°C, 30 sec, 72°C, 30 sec, 40 cycles), the antiviral efficacy of the materials was verified.

As could be seen in [Table 8], it could be confirmed that infection and proliferation of FCV were inhibited, when inosine, xanthosine and guanosine in a dialdehyde form and an acyclic diol form were treated into cells. The guanosine in a dialdehyde form showed the most excellent efficacy (IC₅₀: 18.2 µM), and excellent effects were confirmed in the order of xanthosine and inosine. In addition, the materials in an acyclic diol form also showed the efficacy in the order of guanosine, xanthosine and inosine.

**[Table 8]**

| FCV infection inhibition concentration, cytotoxicity and selectivity index by inosine, xanthosine and guanosine in a dialdehyde form and an acyclic diol form | | | |
|---|---|---|---|
| Classification | IC₅₀ | CC₅₀ | SI |
| Dialdehyde-Inosine | < 26.0 uM (±12.4) | > 10000 uM | > 384.6 |
| Dialdehyde-Xanthosine | < 20.1 uM (±3.4) | > 8000 uM | > 398.0 |
| Dialdehyde-Guanosine | < 18.2 uM (±3.7) | > 6000 uM | > 329.7 |
| Acyclic diol-Inosine | < 362.4 uM (±116.0) | > 10000 uM | > 27.6 |
| Acyclic diol-Xanthosine | < 155.1 uM (±67.2) | > 10000 uM | > 64.5 |
| Acyclic diol-Guanosine | < 133.1 uM (±30.8) | > 10000 uM | > 75.1 |

### Example 9: Feline Infectious peritonitis virus (FIPV)

In order to evaluate viral infection and inhibitory efficacy of inosine, xanthosine, and guanosine in a dialdehyde form and an acyclic diol form against FIPV, feline fetal cells (FCWF-4 cell line) were seeded in a 96 well plate by 2x10⁴ cells/well, and then cultured for one day. Next day, FIPV of 100 TCID₅₀/well and inosine, xanthosine, and guanosine in a dialdehyde form and an acyclic diol form were simultaneously treated into the cells, and after 2 days, RNA was extracted from the infected cells using a commercialized viral RNA extraction kit (Ribospin^{™} vRD II). For the extracted RNA, using FIPV-specific primers (F-TGGCATCTTGCTAACTGGAACT, R-TGCCATAAACGAGCCAGCTA) and realtime RT-qPCR experimental method (95°C, 15 min 1 cycle, 95°C, 30 sec, 58°C, 40 sec, 40 cycles), the antiviral efficacy of the materials was verified.

As could be seen in [Table 9], the inosine in a dialdehyde form showed excellent inhibitory efficacy of infection and proliferation (IC₅₀: 45.5 µM) against FIPV was shown, and excellent effects could be confirmed in the order of guanosine and xanthosine. Nevertheless, the difference between the IC₅₀ values of each material was not big. In addition, in case of the acyclic diol form, antiviral efficacy against FIPV was shown in the order of inosine, xanthosine and guanosine.

**[Table 9]**

| FIPV infection inhibition concentration, cytotoxicity and selectivity index by inosine, xanthosine and guanosine in a dialdehyde form and an acyclic diol form | | | |
|---|---|---|---|
| Classification | IC₅₀ | CC₅₀ | SI |
| Dialdehyde-Inosine | < 45.5 uM (±1.8) | > 10000 uM | > 219.9 |
| Dialdehyde-Xanthosine | < 55.1 uM (±12.7) | > 10000 uM | > 181.4 |
| Dialdehyde-Guanosine | < 52.8 uM (±14.3) | > 6000 uM | > 113.7 |
| Acyclic diol-Inosine | < 872.3 uM (±0) | > 10000 uM | > 11.5 |
| Acyclic diol-Xanthosine | < 1025 uM (±78.5) | > 10000 uM | > 9.8 |
| Acyclic diol-Guanosine | < 1511 uM (±880.4) | > 10000 uM | > 6.6 |

### Example 10: Foot and Mouth disease virus (FMDV)

In order to evaluate inhibitory efficacy of viral infection of inosine and guanosine in a dialdehyde form and inosine, xanthosine and guanosine in an acyclic diol form against a foot and mouth disease virus (FMDV), hamster kidney cells (BHK-21 cell line) were seeded in a 48 well plate by 5x10⁴ cells/well, and then cultured for one day. Next day, FMDV of 0.01 MOI and inosine and guanosine in a dialdehyde form and inosine, xanthosine and guanosine in an acyclic diol form were simultaneously treated into the cells, and after 36 hours, RNA was extracted from the infected cells using a commercialized viral RNA extraction kit (Ribospin^{™} vRD II). For the extracted RNA, using FMDV-specific primers (F-CCGACCCCTCATTCAGCAGACCTC, R-GAGGGTTCTTTTCCGCGTCGCC) and realtime RT-qPCR experimental method (95°C, 5 min 1 cycle, 95°C, 10 sec, 60°C, 30 sec, 40 cycles, 95°C, 15 sec, 60°C, 60 sec, 95°C, 15 sec, 1 cycle melting curve), the antiviral efficacy of the materials was verified.

As could be seen in [Table 10], the guanosine in a dialdehyde form showed very excellent inhibitory efficacy of infection and proliferation (IC₅₀: 114.8 µM) against FMDV was shown. The materials in an acyclic diol form showed antiviral efficacy against FMDV in the order of inosine, xanthosine and guanosine, but showed a slightly higher IC₅₀ value compared to the dialdehyde form.

**[Table 10]**

| FMDV infection inhibition concentration, cytotoxicity and selectivity index by inosine, xanthosine and guanosine in a dialdehyde form and an acyclic diol form | | | |
|---|---|---|---|
| Classification | IC₅₀ | CC₅₀ | SI |
| Dialdehyde-Inosine | < 436.5 uM (±0.037) | > 10000 uM | > 22.9 |
| Dialdehyde-Guanosine | < 114.8 uM (±0.021) | > 6000 uM | > 52.3 |
| Acyclic diol-Inosine | < 1202.3 uM (±0.026) | > 10000 uM | > 8.3 |
| Acyclic diol-Xanthosine | < 1995.3 uM (±0.041) | > 10000 uM | > 5.0 |
| Acyclic diol-Guanosine | < 2060.6 uM (±0.016) | > 10000 uM | > 4.9 |

### Example 11: Evaluation of the immune enhancing efficacy of nucleoside analogues derived from nucleic acid analogs

To confirm the effectiveness of enhancing the expression of Interferon stimulating gene, which is one of the representative antiviral mechanisms of nucleoside analogue (Johnson Y. N. Lau, et al. 2002. Mechanism of action of Ribavirin in the combination treatment of chronic HCV infection. Hepatology. 35(5):1002- 9. Doi: 10.1053/jhep.2002.32672, Paeshuyse J., et al., 2011. Ribavirin for the treatment of chronic hepatitis C virus infection: a review of the proposed mechanism of action. Curr. Opin. Virol. 1: 590- 598. Doi: 10.1016/j.coviro.2011.10.030), inosine, xanthosine and guanosine in the form of dialdehyde, and inosine, xanthosine and guanosine in the form of acyclic diol were used to evaluate the effect of increasing the expression of ISG15, Mx1, and RNaseL, representative interferon stimulating genes that are very important in inducing antiviral immunity.

Pig alveolar macrophages (PAM cell line) were seeded at 1x10⁵ cells/well in a 48 well plate and cultured for one day. The next day, inosine xanthosine and guanosine in the form of dialdehyde, and inosine, xanthosine, and guanosine in the form of acyclic diol were treated with the cells and were extracted from infected cells using the commerciallized easy-spin [DNA free] total RNA extraction kit (iNtRON) 12 hours later. The extracted RNA was quantified for the same gene amount using pig beta-actin (F-GACCACCTTCAACTCGATCA, R-GTGTTGGCGTAGAGGTCCTT), and then realtime RT-Qpcr test (95°C, 5 minutes 1 cycle, 95°C, 10 seconds, 65°C, 30 seconds, 56°C, 60 seconds 40 cycles) by using ISG15, Mx1, RNaseL-specific primers (ISG15: F-GGTGCAAAAGCTTCAGAGACC, R-GTCAGCCAGACCTCATAGGC / Mx1: Real-time RT-qPCR experiment using F-AGCGCAGTGACACCAGCGAC, R-GCCCGGTTCAGCCTGGGAAC / RNaseL: F-GCCAGACCTAGTGGCTTCTG, R-AGAGGCCCAGAGAGTTGTGA) in order to verify the expression rate of immunoregulatory factors of the tested materials.

As shown in [Table 11], it is confirmed that the expression of Interferon stimulating genes (ISG15, Mx1, RNaseL) is increased by inosine, xanthosine, and guanosine in dialdehyde and acyclic diol forms. It is observed that the expression of ISG15 gene increased 4 to 4.7 times, the expression of Mx1 gene increased 3.5 times, and the expression of RNaseL gene increased 2.5 to 3.4 times by dialdehyde forms of inosine, xanthosine, and guanosine. With acyclic diol forms of inosine, xanthosine, and guanosine, the expression of ISG15 gene was increased 4.2-6.3 times, the expression of Mx1 gene was increased 2.8-4.0 times, and the expression of RNaseL gene was increased 1.7-3.0 times. This increase in interferon stimulating genes indicates that materials in the form of dialdehyde and acyclic diol have an immune-enhancing effect to induce antiviral efficacy.

**[Table 11]**

| Interferon stimulating gene expression value (2^{-ΔΔCt} value) by inosine, xanthosine, and guanosine in dialdehyde and acyclic diol forms. | | | |
|---|---|---|---|
| Group | Fold change (multiple increase compared to control group) | | |
| | ISG15 | Mx1 | RNaseL |
| Dialdehyde-Inosine (400 uM) | 4.0 (±0.6) | 3.5 (±1.1) | 3.0 (±0.2) |
| Dialdehyde-Xanthosine (400 uM) | 4.5 (±0.4) | 3.5 (±1.0) | 3.4 (±0.2) |
| Dialdehyde-Guanosine (400 uM) | 4.7 (±0.3) | 3.5 (±0.7) | 2.5 (±0.1) |
| Acyclic diol-Inosine (4000 uM) | 6.3 (±0.4) | 2.8 (±0.9) | 2.0 (±0.3) |
| Acyclic diol-Xanthosine (4000 uM) | 4.7 (±1.3) | 2.7 (±1.4) | 1.7 (±0.3) |
| Acyclic diol-Guanosine (4000 uM) | 4.2 (±1.5) | 4.0 (±1.2) | 3.0 (±0.8) |
| Positive control (INF-β, 20 ng) | 21.1 (±8.81) | 20.0 (±3.1) | 2.9 (±0.9) |

## Claims

1. An antiviral composition comprising at least one nucleoside analogue selected from the group consisting of nucleoside analogues of the following Chemical formulas 1 to 6 and pharmaceutically acceptable salts thereof:

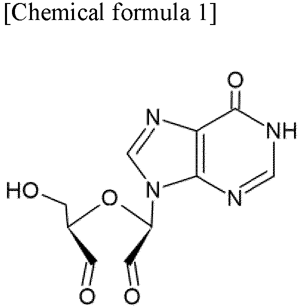

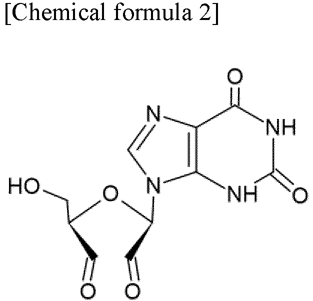

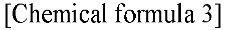

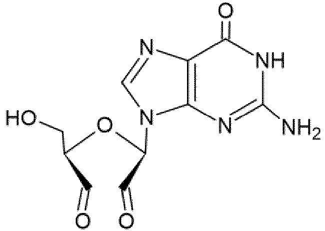

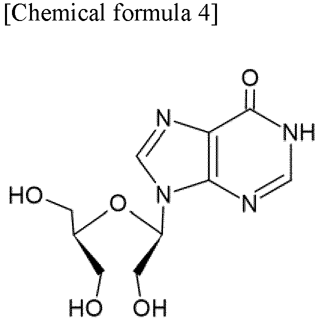

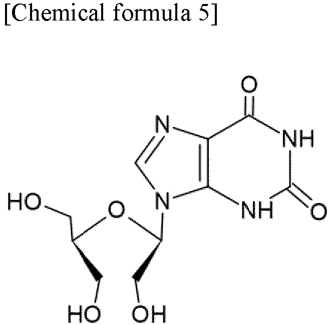

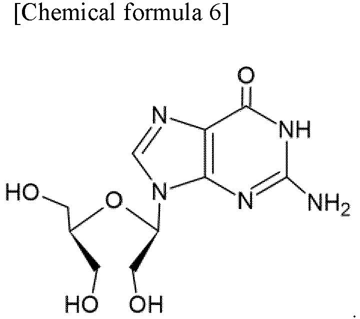

2. The composition according to claim 1, wherein the virus is at least one virus selected from the group consisting of African Swine Fever Virus (ASFV), Classical Swine Fever Virus (CSFV), Low pathogenic Avian influenza virus (LPAIV), Canine coronavirus (CCoV), Canine adenovirus (CAV), Canine distemper virus (CDV), Feline parvovirus (FPV), Feline Calicivirus (FCV), Feline infectious peritonitis virus (FIPV, also known as Feline Coronavirus, FCoV) and Foot and Mouth disease virus (FMDV).

3. An immunomodulatory composition comprising at least one nucleoside analogue selected from the group consisting of nucleoside analogues of the following Chemical formulas 1 to 6 and pharmaceutically acceptable salts thereof:

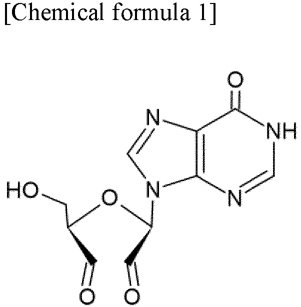

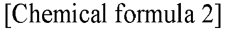

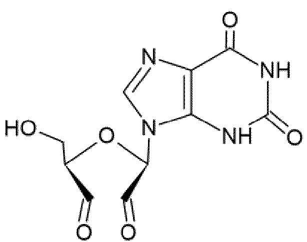

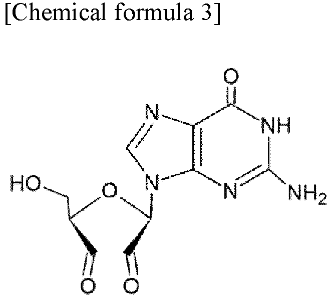

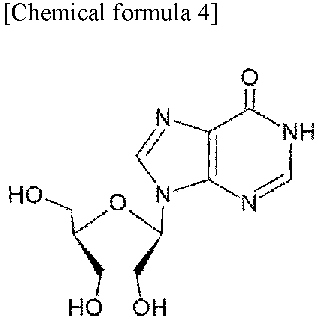

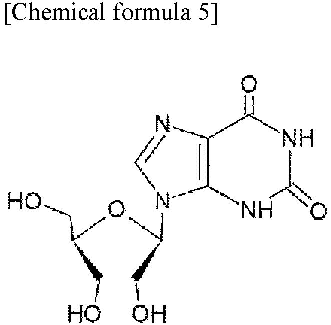

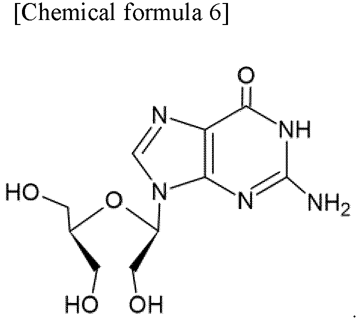

4. The composition according to claim 1 or claim 2, which is a pharmaceutical composition or a food composition.

5. A feed additive comprising at least one nucleoside analogue selected from the group consisting of nucleoside analogues of the following Chemical formulas 1 to 6 and pharmaceutically acceptable salts thereof:

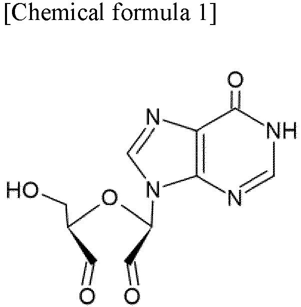

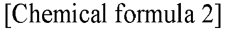

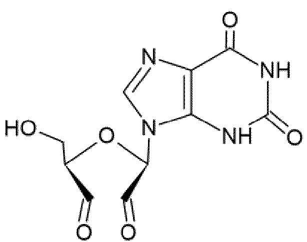

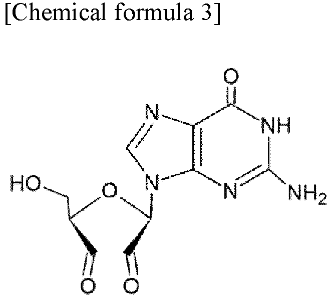

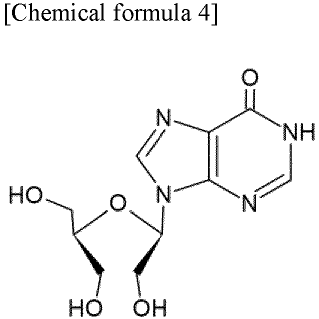

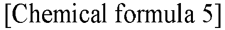

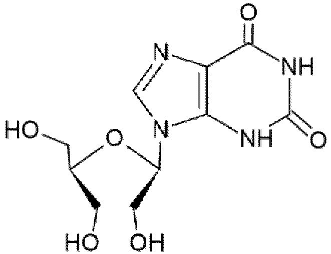

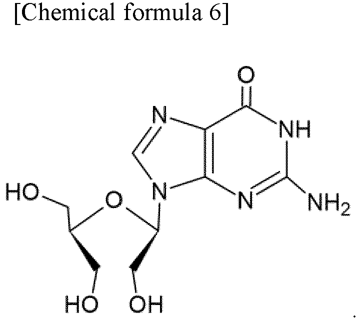

6. The feed additive according to claim 5, which is for mammals, birds, fish or arthropods.

7. A feed comprising at least one nucleoside analogue selected from the group consisting of nucleoside analogues of the following Chemical formulas 1 to 6 and pharmaceutically acceptable salts thereof:

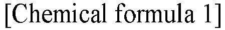

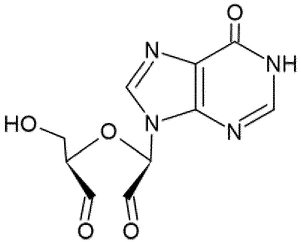

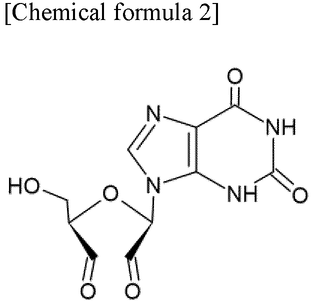

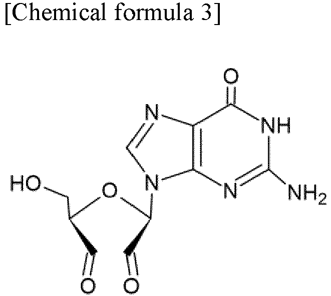

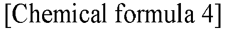

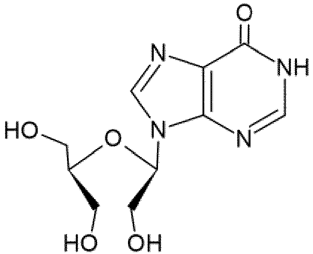

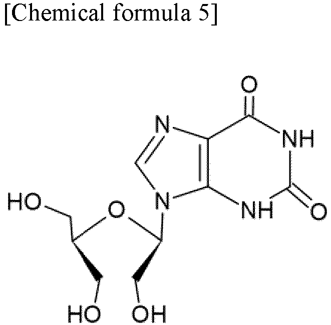

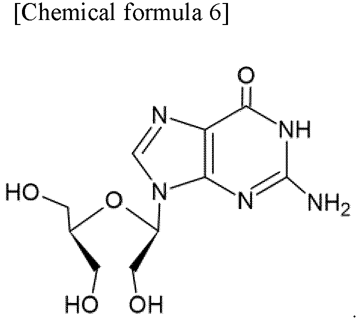

8. The feed according to claim 7, which is for mammals, birds, fish or arthropods.

9. A method for prevention, improvement or treatment of a virus comprising administering at least one nucleoside analogue selected from the group consisting of nucleoside analogues of the following Chemical formulas 1 to 6 and pharmaceutically acceptable salts thereof into a subject:

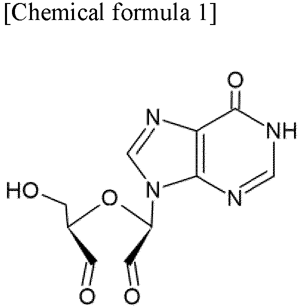

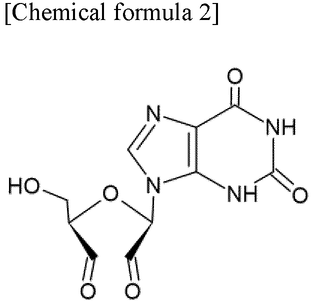

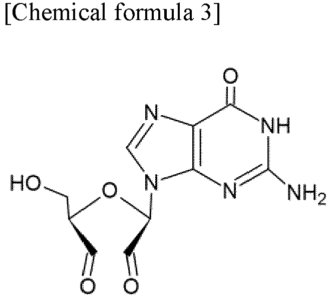

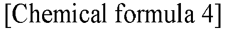

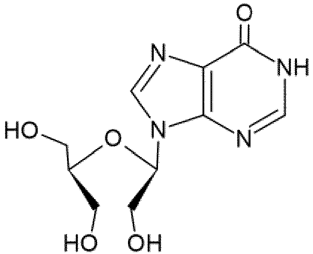

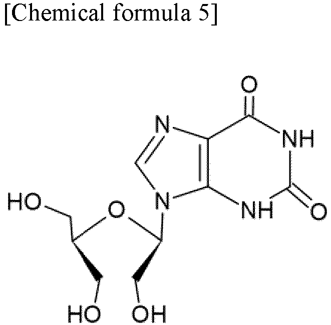

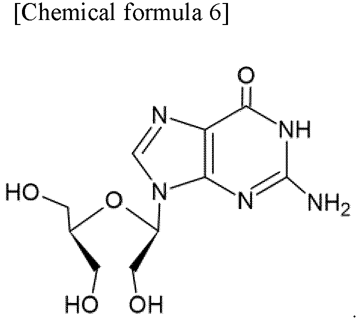

10. A method for modulating immunity comprising administering at least one nucleoside analogue selected from the group consisting of nucleoside analogues of the following Chemical formulas 1 to 6 and pharmaceutically acceptable salts thereof into a subject:
